# EUROPEAN PATENT APPLICATION

(11) **EP 3 919 487 A1**
(43) Date of publication of application: **08.12.2021**
(21) Application number: 19908100.1
(22) Date of filing: 29.01.2019
(51) Int. Cl.: C07D 405/04, C07D 257/02, A61K 49/06

(54) **PREPARATION METHOD AND APPLICATION OF GADOLINIUM IONIC CONTRAST AGENT INTERMEDIATE**

(30) Priority: 28.01.2019 CN 201910078991
(71) Applicant: Hubei Tianshu Pharmaceutical Co., Ltd, Fine Chemicals Industrial Park Yicheng Hubei 441021 (CN)
(72) Inventor: ZHANG, Zhihua, Yicheng, Hubei 441021 (CN); LIU, Yunlong, Yicheng, Hubei 441021 (CN)
(74) Representative: Eisenführ Speiser
(86) International application number: PCT/CN2019/073690
(87) International publication number: WO 2020/154892

(57) **Abstract**

The present invention provides a preparation method and application of an intermediate of gadolinium-based ionic contrast agent. The preparation method comprises the following step: allowing a substance represented by the general formula (II) to react a substance represented by the general formula (III) or a substance represented by the general formula (IV) in the presence of a basic catalyst to generate an intermediate of gadolinium-based ionic contrast agent containing the structure represented by the general formula (I) or the general formula (V), wherein R represents C₁-C₅ alkyl, benzyl or benzyl derivative, R₁ represents -H or -CH₂OH, and R₂ represents -CH₃ or -OH. When preparing an intermediate of gadolinium-based ionic contrast agent having the structure represented by the general formula (I) using the above-mentioned preparation method, it can provide the advantages of simple reaction, fewer steps, controllable reaction, a yield up to 99% or more, and a purity greater than 99.5% for the obtained product.

## Description

### Cross-reference to Related Application

The present application claims the priority to the Chinese patent application No. 201910078991.8 filed on January 28, 2019, the disclosure of which is incorporated herein by reference in its entirety.

### Technical Field

The present invention relates to the technical field of contrast agents for diagnostic imaging, and more particularly, to a preparation method of an intermediate of gadolinium-based ionic contrast agent and use thereof.

### Background Art

Nuclear Magnetic Resonance Imaging (NMRI), also known as Magnetic Resonance Imaging (MRI), has become a conventional diagnostic technology in clinical medical diagnosis due to its advantages such as non-invasiveness, high-resolution anatomical imaging, and non-toxicity and harmlessness to living organisms. NMRI is based on the relaxation of water protons in the human body from high-energy hydrogen nuclei to low-energy nuclei under an external magnetic field to generate image. The image quality is affected by the density and distribution of water protons in the human body. In many cases, it is difficult to reach a clear MRI anatomy map, so it is difficult to accurately judge the disease and injury. The contrast agent can change the relaxation time of the surrounding water protons, increase the magnetic resonance contrast between the detection target site and the surrounding background tissue, and improve the sensitivity and accuracy of NMRI.

So far, gadolinium-centered-complex-based targeting magnetic resonance technology has been most widely used in clinic. The outer layer of gadolinium (Gd) has 7 unpaired electrons, so it is a powerful paramagnetic ion. Non-complexed gadolinium ions are toxic, but the toxic effect of gadolinium can be almost completely eliminated without significantly affecting its paramagnetic effect after the gadolinium forms a complex with a non-toxic organic chelating agent. Various chelating agents of gadolinium that can be injected intravenously have become an integral part of magnetic resonance imaging (MRI) technology, such as gadobutrol or gadoteridol, wherein gadobutrol is a non-ionic complex composed of gadolinium (III) and macrocyclic ligand 10-(2,3-dihydroxy-1-(hydroxymethyl)propyl)-1,4,7,10-tetraazacyclododecane-1,4,7-triacetat e (butrol). Gadoteridol injection is a gadolinium-based non-ionic contrast agent, can be injected intravenously and used for MRI contrast examination of brain, spinal cord, liver and the like, and its raw material drug is gadoteridol (also called 10-(2-hydroxypropyl)-1,4,7,10-tetraazacyclododecane-1,4,7-triacetate gadolinium complex).

A variety of methods for preparing gadobutrol and gadoteridol have been described in the prior art. In the prior art, some synthetic routes are only suitable for laboratory scale, and not suitable for large-scale production; and some synthetic routes require a large amount of resin to purify the target product, resulting in high cost, low yield, and low purity when they are applied to large-scale production.

### Summary of the Invention

A first purpose of the present invention is to provide a preparation method of an intermediate of gadolinium-based ionic contrast agent. The reaction equation of the preparation method is as follows: 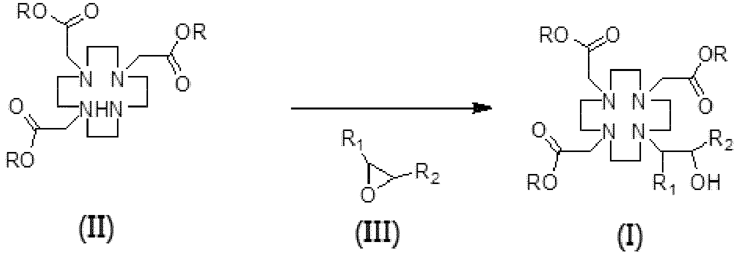 or

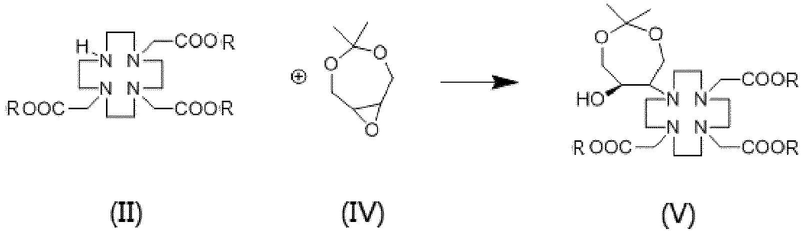

The preparation method comprises the following step:
subjecting a substance represented by the general formula (II) and a substance represented by the general formula (III) or a substance represented by the general formula (IV) to reaction in the presence of a basic catalyst to generate an intermediate of gadolinium-based ionic contrast agent, which has the structure represented by the general formula (I) or the general formula (V);

In the above general formula, R represents C₁-C₅ alkyl, benzyl or benzyl derivative, R₁ represents -H or -CH₂OH, and R₂ represents -CH₂OH, -CH₃ or -OH.

In a preferred embodiment of the present invention, in the general formula (I), R represents C₁-C₅ alkyl, R₁ represents -H or -CH₂OH, and R₂ represents -CH₂OH, -CH₃ or -OH. More preferably, in the general formula (I), R represents tert-butyl, R₁ represents -H, and R₂ represents -CH₃, or R represents tert-butyl, R₁ represents -CH₂OH, and R₂ represents -CH₂OH.

In a preferred embodiment of the present invention, in the general formula (V), R represents C₁-C₅ alkyl, preferably tert-butyl.

In a preferred embodiment of the present invention, the basic catalyst is an inorganic basic catalyst or an organic basic catalyst; the inorganic basic catalyst is one or more selected from potassium carbonate, sodium carbonate, lithium carbonate, potassium bicarbonate, sodium bicarbonate and lithium bicarbonate; the organic basic catalyst is an organic amine catalyst, and the organic amine catalyst is one or more selected from trimethylamine, tripropylamine, tributylamine, dimethylaniline, diethylaniline, pyridine, pyridine derivative and compounds similar to the above-mentioned structures. More preferably, the inorganic basic catalyst is one or more selected from potassium carbonate, sodium carbonate and lithium carbonate, most preferably potassium carbonate.

In a preferred embodiment of the present invention, the mass ratio of the substance represented by the general formula (II), the substance represented by the general formula (III) and the basic catalyst is (10-100) : (1-10) : 1, preferably (11-20) : (1-10) : 1, preferably 13:5:1.

In a preferred embodiment of the present invention, the reaction temperature is 40 to 180°C, and the reaction time is 6 to 18 h; preferably, the reaction temperature is 60°C, and the reaction time is 12 h.

In a preferred embodiment of the present invention, toluene or isopropanol can be used as a solvent in the above two reactions, wherein, the solvent is added in an amount enough to dissolve the substrate; wherein, when R represents tert-butyl, R₁ represents -H, and R₂ represents -CH₃ in the formula (I), toluene can be used as a solvent for the reaction; and when R represents tert-butyl, R₁ represents -CH₂OH, and R₂ represents -OH, isopropanol can be used as a solvent for the reaction.

After the reaction is finished, the resulting reaction solution may be washed with water and dried, and the solvent is removed to obtain a high-purity intermediate of gadolinium-based ionic contrast agent, which has the structure represented by the general formula (I) or the general formula (V).

Another purpose of the present invention is to provide an intermediate of gadolinium-based ionic contrast agent having the structure represented by the general formula (I) or general formula (V) obtained by the above preparation method, that is, the intermediate of gadolinium-based ionic contrast agent provided has the structure represented by general formula (I) or general formula (V): 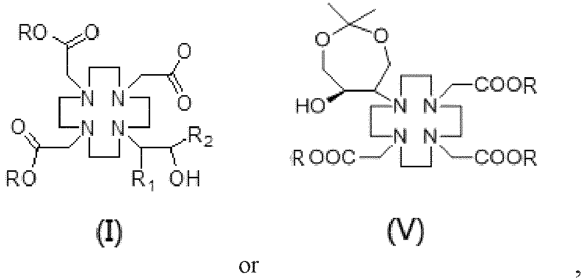 wherein, R is preferably tert-butyl. R₁ represents -H, R₂ represents -CH₃, or both R₁ and R₂ represent -CH₂OH.

When the above-mentioned preparation method is used to prepare an intermediate of gadolinium-based ionic contrast agent, which has a structure represented by the general formula (I), the yield is up to 98% or more, and the purity of the obtained product is greater than 99%, preferably greater than 99.5%.

Another purpose of the present invention is to provide use of the above preparation method in preparing a gadolinium-based ionic contrast agent, that is, a preparation method of a gadolinium-based ionic contrast agent is provided. The preparation method comprises the following steps: hydrolyzing the intermediate of gadolinium-based ionic contrast agent, and forming a salt to obtain the gadolinium-based ionic contrast agent;
wherein, the hydrolyzing step may adopt acid hydrolysis commonly used in the art. Preferably, the hydrolyzing step may comprise hydrolyzing the intermediate of gadolinium-based ionic contrast agent in the presence of a catalyst, and the preparation method of the catalyst comprises the following steps: subjecting zirconia and titanium tetrachloride to reaction in the presence of sulfuric acid and water at 60°C to 90°C until the solids are dissolved, adding silica to perform reaction for 1 to 5 h, filtering to obtain the solid, washing and calcining the solids;
wherein, the molar ratio of the zirconia to titanium tetrachloride is preferably 1 : (0.1-1), and more preferably 1 : (0.4-0.5);
wherein, the volume ratio of the sulfuric acid to water is preferably (0.5-3) : 10, and more preferably (1-1.5) : 10;
wherein, the molar ratio of the zirconia to silica is 1 : (10-20), and preferably 1 : (12-15);
wherein, the calcination temperature is 500°C to 700°C, and preferably 550°C to 600°C.

In a preferred embodiment of the present invention, the mass ratio of the tert-butyl ester to the catalyst is 1 : (1-1000), preferably 1 : (50-100), and more preferably 1:100;
wherein, the hydrolysis temperature is 70°C to 90°C and the hydrolysis time is 8 to 24 h; preferably, the hydrolysis temperature is 85°C to 90°C and the hydrolysis time is 12 to 15 h.

In the above hydrolysis reaction, the weight ratio of the added water to the tert-butyl ester is 20:1 to 20:5.

In a preferred embodiment of the present invention, the hydrolyzing step may specifically comprise: mixing the above-mentioned intermediate of gadolinium-based ionic contrast agent with water, adding the above-mentioned catalyst, and reacting at 70°C to 90°C for 8 to 24 h.

The hydrolyzing step may further comprise, after the end of the hydrolysis reaction, filtering the reaction solution, washing with water, concentrating, and then recrystallizing using ethanol and/or acetone, to give a product for use in the subsequent salt forming reaction.

In a preferred embodiment of the present invention, when the intermediate has the structure represented by the general formula (I), in which R represents tert-butyl, R₁ represents -H, and R₂ represents -CH₃, ethanol can be used as a solvent for recrystallization. When R represents tert-butyl, R₁ represents -CH₂OH, and R₂ represents -OH, a mixed solvent of ethanol and acetone can be used as a solvent for recrystallization, wherein the volume ratio of ethanol to acetone is preferably (5.5-7) : (3-5), and more preferably 6:4.

A person skilled in the art can prepare a gadolinium-based ionic contrast agent by using the salt-forming reaction commonly used in the art. In the embodiment of the present invention, the salt-forming step may specifically comprise:
dissolving the hydrolyzed product in water, adding gadolinium trioxide, reacting at 60 to 100°C until the solution is clear, and recrystallizing to obtain the gadoteridol.
wherein, the molar ratio of the hydrolyzed product to gadolinium trioxide is preferably 1:1.1 to 0.9:1, and more preferably 1:1.02 to 0.95:1;
wherein, the reaction is preferably performed at 85°C until the solution is clear;
wherein, isopropanol is preferably used for recrystallization, and the mass ratio of the isopropanol to teridol is preferably 3:1 to 20:1.

When preparing an intermediate of gadolinium-based ionic contrast agent having the structure represented by the general formula (I) or the general formula (V) using the above-mentioned preparation method, it can provide the advantages of simple reaction, fewer steps, controllable reaction, a yield up to 98% or more, and a purity greater than 99% for the obtained product. The intermediate of the present invention is used to prepare a gadolinium-based ionic contrast agent. The preparation method provided has high yield, high product purity, and less waste gas, waste water and waste residues generated during the preparation process, which can effectively reduce environmental pollution.

### Specific Modes for Carrying Out the Embodiments

The specific embodiments of the present invention will be described in further detail in combination with the Examples below. The following Examples are used to illustrate the present invention, but not to limit the scope of the present invention.

Unless otherwise specified, the technical means used in the Examples are conventional means well known to a person skilled in the art, and the raw materials used are all commercially available products.

### Example 1

The present Example provides a preparation method of an intermediate of gadolinium-based ionic contrast agent. The reaction equation for the preparation method is as follows: 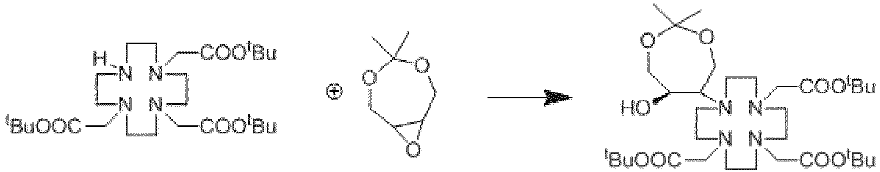 the preparation method comprises the following steps:
26 g of tert-butyl 1,4,7,10-tetraazacyclododecane-1,4,7-triacetate, 2 g of potassium carbonate, 300 mL of isopropanol, and 10 g of 4,4-dimethyl-3,5,8-trioxabicyclo[5,1,0]octane were added into a 1 L reaction flask to perform reaction at 60°C for 12 h, then isopropanol was recovered, 300 mL of toluene was added, the resulting reaction solution was washed with water, dried with anhydrous magnesium sulfate, and then toluene was recovered under reduced pressure to obtain 33 g of a light yellow oily substance with a yield greater than 99% and a purity greater than 99.8%.

The elemental analysis results of the light yellow oily substance obtained in the present Example were as follows: C: 60.12%, N: 8.48%, and H: 9.46% (C₃₃H₆₂N₄O₉ theoretical values: C: 60.18%, H: 9.43%, and N: 8.51%). From the above data, it can be proved that the above intermediate was successfully synthesized in the present Example.

### Example 2

The present Example provides a preparation method of an intermediate of gadolinium-based ionic contrast agent. The reaction equation of the preparation method is as follows: 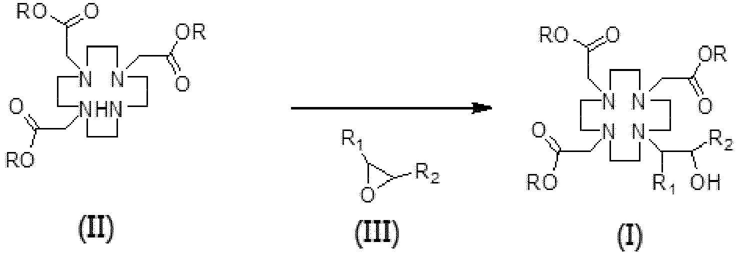 the preparation method comprises the following steps:
30 g of tert-butyl 1,4,7,10-tetraazacyclododecane-1,4,7-triacetate hydrobromide, 20 g of potassium carbonate, 300 mL of toluene, 10 mL of propylene oxide were added into a 1 L reaction flask to perform reaction at 60°C for 12 h, then the resulting reaction solution was washed with water, dried with anhydrous magnesium sulfate, and toluene was recovered under reduced pressure to obtain 28 g of a light yellow oily substance with a yield greater than 99% and a purity greater than 99.5%;
wherein, in the intermediate of gadolinium-based ionic contrast agent (I) obtained in the present Example, R represents tert-butyl, R₁ represents -H, and R₂ represents -CH₃.

The elemental analysis results of the light yellow oily substance obtained in the present Example were as follows: C: 59.76%, N: 9.62%, and H: 9.79% (C₂₉H₅₆N₄O₇ theoretical values: C: 59.97%, N: 9.65%, and H: 9.72%). From the above data, it can be proved that the above intermediate was successfully synthesized in the present Example.

### Example 3

The present Example provides a preparation method of an intermediate of gadolinium-based ionic contrast agent. The reaction equation is the same as that in Example 1. The preparation method comprises the following steps:
26 g of tert-butyl 1,4,7,10-tetraazacyclododecane-1,4,7-triacetate, 2 g of sodium carbonate, 300 mL of isopropanol, and 10 g of 4,4-dimethyl-3,5,8-trioxabicyclo[5,1,0]octane were added into a 1 L reaction flask to perform reaction at 40°C for 18 h, then isopropanol was recovered, 300 mL of toluene were added, the resulting reaction solution was washed with water, dried with anhydrous magnesium sulfate, and then toluene was recovered under reduced pressure to obtain a light yellow oily substance with a yield greater than 98.6% and a purity greater than 99.5%.

The elemental analysis results of the light yellow oily substance obtained in the present Example were the same as those of the product obtained in Example 1, which can prove that the above intermediate was successfully synthesized in the present Example.

### Example 4

The present Example provides a preparation method of an intermediate of gadolinium-based ionic contrast agent. The reaction equation is the same as that in Example 1. The preparation method comprises the following steps:
40 g of tert-butyl 1,4,7,10-tetraazacyclododecane-1,4,7-triacetate, 2 g of dimethyl aniline, 300 mL of isopropanol, and 20 g of 4,4-dimethyl-3,5,8-trioxabicyclo[5,1,0]octane were added into a 1 L reaction flask to perform reaction at 180°C for 4 h, then isopropanol was recovered, 300 mL of toluene was added, the resulting reaction solution was washed with water, dired with anhydrous magnesium sulfate, and then toluene was recovered under reduced pressure to obtain a light yellow oily substances with a yield greater than 98.2% and a purity greater than 99.3%.

The elemental analysis result of the light yellow oily substances obtained in the present Example is the same as that of the product obtained in Example 1, which can prove that the above intermediate was successfully synthesized in the present Example.

### Example 5

The present Example provides a preparation method of an intermediate of gadolinium-based ionic contrast agent. The reaction equation is the same as that in Example 1. The preparation method comprises the following steps:
100 g of tert-butyl 1,4,7,10-tetraazacyclododecane-1,4,7-triacetate, 1 g of potassium carbonate, 300 mL of isopropanol, and 10 g of 4,4-dimethyl-3,5,8-trioxabicyclo[5,1,0]octane were added into a 1 L reaction flask to perform reaction at 60°C for 12 h, then isopropanol was recovered, 300 mL of toluene was added, the resulting reaction solution was washed with water, dried with anhydrous magnesium sulfate, and then toluene was recovered under reduced pressure to obtain a light yellow oily substance with a yield greater than 98% and a purity greater than 99%.

The elemental analysis results of the light yellow oily substance obtained in the present Example were the same as those of the product obtained in Example 1, which can prove that the above intermediate was successfully synthesized in the present Example.

### Example 6

The present Example provides a preparation method of an intermediate of gadolinium-based ionic contrast agent, and the reaction equation is the same as that in Example 2. The preparation method comprises the following steps:
30 g of tert-butyl 1,4,7,10-tetraazacyclododecane-1,4,7-triacetate hydrobromide, 20 g of trimethylamine, 300 mL of toluene, and 10 mL of propylene oxide were added into a 1 L reaction flask to perform reaction at 60°C for 12 h, then the resulting reaction solution was washed with water, dried with anhydrous magnesium sulfate, and then toluene was recovered under reduced pressure to obtain 28 g of a light yellow oily substance with a yield greater than 98% and a purity greater than 99%;
wherein, in the intermediate of gadolinium-based ionic contrast agent (I) obtained in the present Example, R represents tert-butyl, R₁ represents -H, and R₂ represents -CH₃.

The elemental analysis results of the light yellow oily substances obtained in the present Example were the same as those of the product obtained in Example 2, which can prove that the above intermediate was successfully synthesized in the present Example.

### Example 7

The present Example provides a preparation method of a gadolinium-based ionic contrast agent. The reaction equation is as follows:

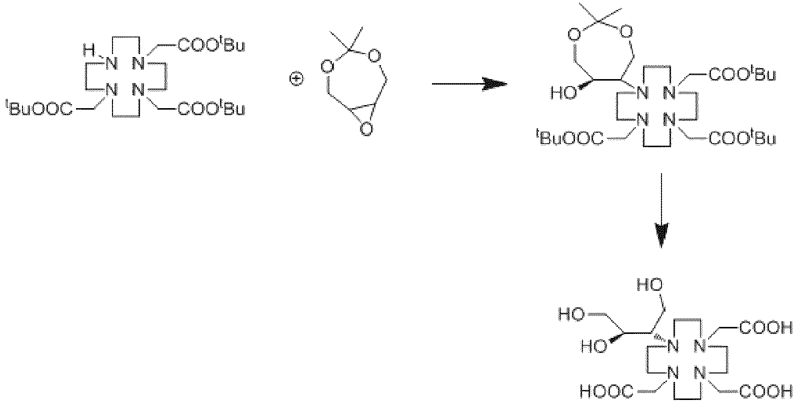

The preparation method comprises the following steps:
1) the intermediate of gadolinium-based ionic contrast agent obtained in Example 1 was added into a 500 mL reaction flask containing 200 mL distilled water, and 0.5 g of a catalyst was added under stirring to perform reaction at 85 to 90°C for 12 h, the resultant was filtered, washed with a small amount of water, concentrated to dry under reduced pressure, and recrystallized with ethanol/acetone (6:4) to obtain 21 g white solid of butrol (the elemental analysis results were as follows: C: 47.82%, N: 12.32%, and H: 7.64%, C₁₈H₃₄N₄O₉ theoretical values: C: 47.97 %, N: 12.43%, and H: 7.62%);
   wherein, the catalyst was prepared by the following method: 5 g of ZrO₂ and 3 g of TiCL₄ were added to 100 mL of distilled water, and 10 mL of sulfuric acid was added, the mixture was heated at about 80°C until the solids were completely dissolved, and then 60 ml of SiO₂ were added to perform adsorption for 2 h, the resultant was filtered, washed twice with water, washed twice with 50 ml of 10% NaOH, and washed with distilled water to neutrality, and then calcined at 550°C to obtain the catalyst;
2) the above-mentioned butrol was dissolved in 100 mL of distilled water, and 9.0 g of Gd₂O₃ was added to perform reaction at 85°C until the solution was clear, then the reaction solution was filtered, concentrated, and recrystallized with 300 mL of 95% ethanol to obtain 28 g white crystal of gadobutrol with a yield greater than 99% and a purity greater than 99.5%;
   wherein, the infrared spectrum (KBr, cm⁻¹) of the white crystal was provided as follows: 3560, 3280, 2975, 2940, 2920, 2880, 2870, 1650, 1600, 1380. It can be proved that the white crystal is gadobutrol.

### Example 8

The present Example provides a preparation method of a gadolinium-based ionic contrast agent. The reaction equation is as follows:

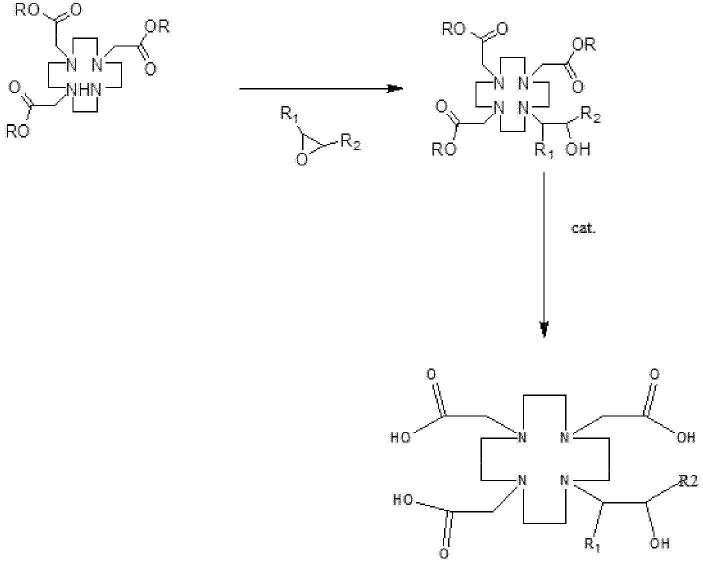

The preparation method comprises the following steps:
1) the intermediate of gadolinium-based ionic contrast agent obtained in Example 2 was added into a 500 mL reaction flask containing 200 mL distilled water, and 0.5 g of a catalyst was added under stirring to perform reaction at 85 to 90°C for 12 h, the resultant was filtered, washed with a small amount of water, concentrated to dry under reduced pressure, and recrystallized with 95% ethanol to obtain 17 g white solid of teridol (the elemental analysis results were as follows: C: 50.37%, N: 13.80%, and H: 8.09%, C₁₇H₃₂N₄O₇ theoretical values: C: 50.44%, N: 13.85 %, and H: 8.01%);
   wherein, the catalyst was prepared by the following method: 5 g of ZrO₂ and 3 g of TiCL₄ were added to 100 mL of distilled water, and 10 mL of sulfuric acid was added, the mixture was heated at about 80°C until the solids were completely dissolved, and then 60 ml of SiO₂ was added to perform adsorption for 2 h, the resultant was filtered, washed twice with water, washed twice with 50 mL of 10% NaOH, and washed with distilled water to neutrality, and then calcined at 550°C to obtain the catalyst;
2) the above-mentioned teridol was dissolved in 100 mL of distilled water, and 9.0 g of Gd₂O₃ was added to perform reaction at 85°C until the solution was clear, then filtered, concentrated, and recrystallized with 200 mL of isopropanol to obtain 28 g white crystal of gadoteridol with a yield greater than 99% and a purity greater than 99.5%.

Finally, the methods described in the examples are only preferred embodiments, and are not intended to limit the protection scope of the present invention. Any modification, equivalent replacement, or improvement made within the spirit and principle of the present invention shall be included in the protection scope of the present invention.

### Industrial applicability

The present invention provides a preparation method and use of an intermediate of gadolinium-based ionic contrast agent. The preparation method includes the following steps: allowing a substance represented by the general formula (II) to react with a substance represented by the general formula (III) or a substance represented by the general formula (IV) in the presence of a basic catalyst to generate an intermediate of gadolinium-based ionic contrast agent having the structure represented by the general formula (I) or the general formula (V), wherein R represents C₁-C₅ alkyl, benzyl or benzyl derivative, R₁ represents -H or -CH₂OH, and R₂ represents -CH₃ or -OH. The preparation method provided by the present invention can be used for industrial large-scale production, and makes up for the shortcomings of the existing method such as suitability for small-scale production only, insufficient purity, and low yield.

## Claims

1. A preparation method of an intermediate of gadolinium-based ionic contrast agent, wherein, the reaction equation for the preparation method is as follows: 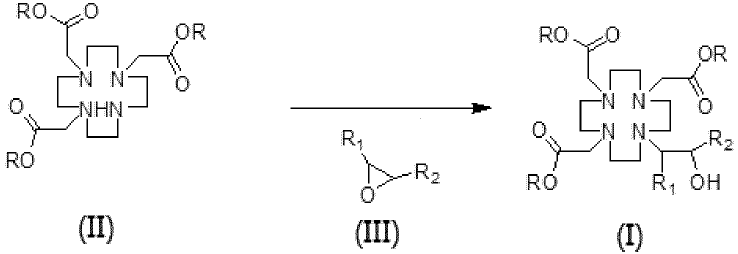 or 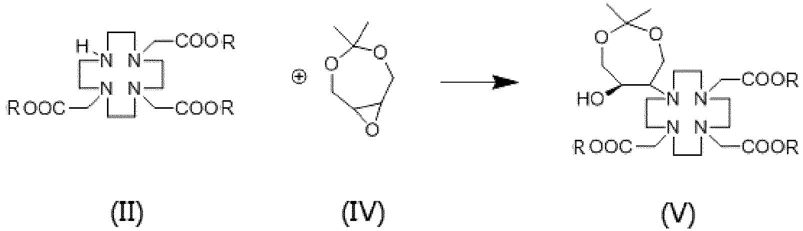 the preparation method comprises the following step:
allowing a substance represented by the general formula (II) to react with a substance represented by the general formula (III) or a substance represented by the general formula (IV) in the presence of a basic catalyst to generate an intermediate of gadolinium-based ionic contrast agent having the structure represented by the general formula (I) or the general formula (V);
wherein, R represents C₁-C₅ alkyl, benzyl or benzyl derivative, R₁ represents -H or -CH₂OH, and R₂ represents -CH₂OH, -CH₃ or -OH.

2. The intermediate of gadolinium-based ionic contrast agent according to claim 1, wherein R represents C₁-C₅ alkyl, R₁ represents -H or -CH₂OH, and R₂ represents -CH₂OH, -CH₃ or -OH.

3. The intermediate of gadolinium-based ionic contrast agent according to claim 1, wherein R represents tert-butyl, R₁ represents -H, and R₂ represents -CH₃, or R represents tert-butyl, R₁ represents -CH₂OH, and R₂ Represents -CH₂OH.

4. The preparation method according to any one of claims 1 to 3, wherein the basic catalyst is an inorganic basic catalyst or an organic basic catalyst; the inorganic basic catalyst is one or more selected from potassium carbonate, sodium carbonate, lithium carbonate, potassium bicarbonate, sodium bicarbonate and lithium bicarbonate; the organic basic catalyst is an organic amine catalyst, and the organic amine catalyst is one or more selected from trimethylamine, tripropylamine, tributylamine, dimethylaniline, diethylaniline, pyridine, pyridine derivative and compounds similar to the above-mentioned structures; further preferably one or more selected from potassium carbonate, sodium carbonate and lithium carbonate, and most preferably potassium carbonate.

5. The preparation method according to any one of claims 1 to 4, wherein the mass ratio of the substance represented by the general formula (II), the substance represented by the general formula (III) or the general formula (IV), and the basic catalyst is (10-100) : (1-10) : 1, preferably 13:5:1.

6. The preparation method according to any one of claims 1 to 5, wherein the reaction temperature is 40 to 180°C and the reaction time is 6 to 18 h; preferably, the reaction temperature is 60°C, and the reaction time is 12 h.

7. The preparation method according to any one of claims 1 to 6, wherein toluene or isopropanol is used as a solvent in the reaction.

8. An intermediate of gadolinium-based ionic contrast agent, wherein, the intermediate has a structure represented by the general formula (V):

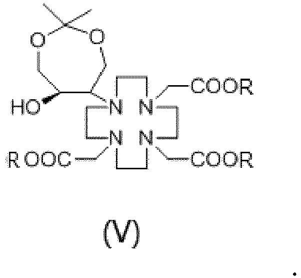

9. Use of the preparation method according to any one of claims 1 to 8 in the preparation of a gadolinium-based ionic contrast agent, wherein the intermediate of gadolinium-based ionic contrast agent is subjected to hydrolysis and prepared into salt to obtain the gadolinium-based ionic contrast agent.

10. The use according to claim 9, wherein the hydrolysis specifically comprises: hydrolyzing the intermediate of gadolinium-based ionic contrast agent in the presence of a catalyst; wherein
the preparation method of the catalyst comprises the following steps: subjecting zirconia and titanium tetrachloride to reaction in the presence of sulfuric acid and water at 60°C to 90°C until the solids are dissolved, adding silica to perform reaction for 1 to 5 h, filtering to obtain solids, washing and calcining the solids.
